(19)
Europäisches Patentamt
European Patent Office
Office européen des brevets

(11) EP 1 275 385 A1

(12) **EUROPEAN PATENT APPLICATION**
published in accordance with Art. 158(3) EPC

(43) Date of publication:
**15.01.2003 Bulletin 2003/03**

(21) Application number: **01919785.4**

(22) Date of filing: **05.04.2001**

(51) Int Cl.[7]: **A61K 31/166**, A61P 9/10, A61P 9/08

(86) International application number:
**PCT/JP01/02946**

(87) International publication number:
**WO 01/076587 (18.10.2001 Gazette 2001/42)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **06.04.2000 JP 2000104344**

(71) Applicant: **ONO PHARMACEUTICAL CO., LTD.**
**Osaka-shi, Osaka 541-8526 (JP)**

(72) Inventors:
- **MATSUNO, Hiroyuki**
  **Gifu 500-8361 (JP)**
- **UEMATSU, Toshihiko**
  **Shizuoka 432-8002 (JP)**

(74) Representative: **Henkel, Feiler, Hänzel**
**Möhlstrasse 37**
**81675 München (DE)**

(54) **REMEDIES FOR DISEASES DUE TO STENOTIC LESIONS OF BLOOD VESSEL**

(57) An agent for treating and/or preventing diseases due to a constrictive vascular lesion, which comprises, as an active ingredient, a hydroxamic acid derivative represented by formula (I):

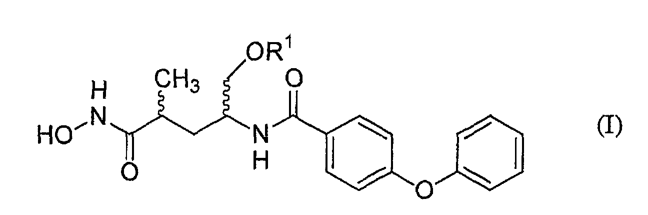

(I)

(wherein $R^1$ represents a hydrogen atom, C1-8 alkyl, or C1-8 alkyl substituted with $-OR^2$; and $R^2$ represents a hydrogen atom, C1-8 alkyl, benzyl, or C1-8 alkyl substituted with C1-8 alkoxy) having a matrix metalloproteinase inhibitory activity, or a nontoxic salt thereof.

EP 1 275 385 A1

## Description

### TECHNICAL FIELD

The present invention relates to an agent for treating and/or preventing diseases due to a constrictive vascular lesion.

More particularly, the present invention relates to an agent for treating and/or preventing diseases due to a constrictive vascular lesion, which comprises, as an active ingredient, a hydroxamic acid derivative represented by formula (I):

(I)

(wherein all symbols have the same meanings as described below) having a matrix metalloproteinase inhibitory activity or a nontoxic salt thereof.

### BACKGROUND ART

A matrix metalloproteinase (hereinafter referred to as "MMP") is a neutral metalloproteinase having zinc in the active site. Up to now, at least 20 subtypes each having a different primary structure have been identified. Examples include interstitial collagenase (MMP-1), leukocyte collagenase (MMP-8), collagenase-3 (MMP-13), gelatinase A (MMP-2), gelatinase B (MMP-9), stromelysin-1 (MMP-3), stromelysin-2 (MMP-10), matrilysin (MMP-7), metalloelastase (MMP-12) and the like.

MMP has effects on growth and tissue remodeling of articulation tissue, bone tissue and connective tissue by degrading collagen, laminin, proteoglycan, fibronectin, elastin, gelatin and the like under physiological conditions. However, it is considered that destruction of various tissues under morbid conditions is caused by abnormal increase in the expression or activity of MMP due to breakdown of the regulation of MMP.

On the other hand, it is considered that stricture of blood vessels is caused by thrombus formation or new inner membrane formation after vascular endothelium injury. More specifically, it is considered that stricture of a blood vessel occurs as a result of thrombus formation due to adhesion of platelets to the blood vessel wall or of new inner membrane formation due to production of growth factors and subsequent migration and growth of smooth muscle cells, induced by a vascular endothelium injury.

There are various diseases which are due to a constrictive vascular lesion. Examples include restenosis after blood vessel injuries by PTCA (percutaneous transluminal coronary angioplasty), *etc.*, unstable angina, acute myocardial infarction, transient cerebral ischemic attack, chronic arterial obstruction and the like.

In most cases, angina is based on the atherosclerosis of coronary arteries and induced by becoming a topically ischemic state due to unbalanced amount of required and supplied oxygen in cardiac muscle. The angina is classified into stable angina and unstable angina depending on its attacking mode, and the unstable angina means an angina relapsed after a silent period of 6 months or more, an exertional angina newly occurred within 3 to 4 weeks and the like. Also, it is considered that from 50 to 80% of them sift over to acute myocardial infarction.

In the acute myocardial infarction conditions, the morbid state of angina further progresses such that blood vessels are blocked, blood flow is stopped, oxygen and nutrition cannot be transferred to cardiac muscle and a part thereof causes necrosis.

Transient cerebral ischemic attack shows the same symptoms of cerebral infarction but its conditions are transient. It is considered that the cause of the attack particularly occurred in the internal carotid area is microembolism generated by the peeling of a part of platelet plug formed in the atherosclerosis region.

As a representative disease of the chronic arterial obstruction, arteriosclerosis obliterans (ASO) is exemplified. Its onset is based on arteriosclerosis and it shows ischemic conditions accompanied by blood flow reduction caused by stricture and obstruction of arteries.

Recently, PTCA has been established as a therapeutic method having a high success ratio for serious constrictive vascular lesions and has been broadly used. This is a therapeutic method in which a stricture-caused blood

vessel induced by angina, myocardial infarction or the like is widened by swelling the blood vessel using a balloon catheter. However, its most serious problem is that restenosis is recognized in 30 to 40% of the succeeded cases by PTCA. It is considered that this restenosis is induced by the thickening of blood vessels which is caused by the thrombus formation after blood vessel injury with the balloon and subsequent production of extracellular matrixes, such as collagen, proteoglycan and the like, induced by the migration and growth of smooth muscle cells. Also, it has been recently found that a blood vessel once widened with a balloon contract chronically. However, this serious problem has not been solved yet, and its therapeutic and/or preventive method has not been established.

At present, MMP's relationship with arteriosclerosis and blood vessel thickening is being revealed. For example, expression of MMP-1, 2, 3 and 9 in human atherosclerosis plaque has been reported [*J. Clin. Invest., 94*(69): 2493-503 (1994); *Proc. Natl. Acad. Sci*., *88*(18): 8154-8 (1991); *Circulation, 91*(8): 2125-31 (1995)], and over-expression of MMP-12 was found in a arteriosclerosis focus through a study using an artery of rabbit loaded with cholesterol feed [*Am. J. Pathol*., *153*(1): 109-119 (1998)]. Also, there is a report showing that expression of MMP-2 and 9 and increase in their activities are related to the migration and growth of smooth muscle cells after blood vessel injury [*Cir. Res*., *75*(3): 539-45 (1994)].

Moreover, several reports have been published on the relationship with MMP inhibitors. For example, it has been reported that GM6001 [3-(N-hydroxycarbamoyl)-2(S)-isobutylpropionyl-L-tryptophan methylamide] inhibits migration of smooth muscle cells [*Circ. Res*., *78*: 38-43 (1996)], and that BB94 [Batimastst; 3(S)-(N-hydroxycarbamoyl)-2(R)-isobutyl-4-(2-thienylthio)butynyl-L-phenylalanine methylamide] inhibits migration and growth of smooth muscle cells dose-dependently, which suggests that BB94 inhibits inner membrane thickening after artery injury [*Arterioscler. Thromb. Vasc. Biol*., *78*: 38-43 (1996)].

DISCLOSURE OF THE INVENTION

Recently, agents for treating and/or preventing diseases due to a constrictive vascular lesion have been desired, and it is expected that an MMP inhibitor will become an agent for treating and/or preventing them. However, all of the already known MMP inhibitors are not always effective for diseases due to a constrictive vascular lesion, and the realization has not been attained yet at present.

Under such circumstances, the present inventors have conducted intensive studies and found for the first time that hydroxamic acid derivatives represented by formula (I) and nontoxic salts thereof are effective on diseases due to a constrictive vascular lesion, and thus the present invention has been accomplished.

Specifically, the present invention relates to an agent for treating and/or preventing diseases due to a constrictive vascular lesion, which comprises, as an active ingredient, a hydroxamic acid derivative represented by formula (I):

(I)

(wherein $R^1$ represents a hydrogen atom, C1-8 alkyl, or C1-8 alkyl substituted with $-OR^2$; and $R^2$ represents a hydrogen atom or C1-8 alkyl) or a nontoxic salt thereof.

The hydroxamic acid derivatives represented by formula (I) are compounds disclosed in WO 99/19296 as compounds having an MMP inhibitory activity.

In WO 99/19296, it is described that since aminobutanoic acid derivatives containing the compound represented by formula (I) have an MMP inhibitory activity, they are effective for rheumatoid diseases, arthrosteitis, unusual bone resorption, osteoporosis, periodontitis, interstitial nephritis, arteriosclerosis, pulmonary emphysema, cirrhosis, cornea injury, metastasis of, invasion or growth of tumor cells, autoimmune diseases (Crohn's disease, Sjogren's syndrome), diseases caused by vascular emigration or infiltration of leukocytes, arterialization, multiple sclerosis, aortic aneurysm, endometriosis and the like. However, it is not described that that the compound represented by formula (I) is effective for diseases due to a constrictive vascular lesion.

Based on a report that MMP-2 and 9 relates to the migration and growth of vascular smooth muscle cells, it is expected that a compound having an inhibitory activity for MMP-2 and/or MMP-9 will inhibit blood vessel thickening. Also, based on a report that MMP-12 is over-expressed in arteriosclerosis foci, it is expected that a compound having

an inhibitory activity for MMP-12 would inhibit arteriosclerosis. However, whether or not they actually show the efficacy cannot be found without testing them using a morbid state model of each disease of interest.

The present inventors have tested several MMP inhibitors using a high cholesterol feed-loaded hamster blood vessel stricture model. As a result, only the compound represented by formula (I) inhibited migration and growth of smooth muscle cells and also inhibited blood vessel thickening. This fact has been found for the first time.

Based on the effective results by this experimental model, it is considered that the compound of the present invention represented by formula (I) is effective in treating and/or preventing diseases due to a constrictive vascular iesion, specifically, restenosis after blood vessel injuries by PTCA and the like, as well as unstable angina, acute myocardial infarction, transient cerebral ischemic attack, chronic arterial obstruction and the like, more preferably, it is considered that it is effective for treating and/or preventing restenosis after PTCA.

BRIEF DESCRIPTION OF THE DRAWINGS

Fig. 1 is a graph showing a result of blood vessel thickening inhibition when an active component according to the present invention is administered.

Fig. 2 is a graph showing a result of smooth muscle cell growth inhibition when an active component according to the present invention is administered.

Fig. 3 is a graph showing a result of smooth muscle cell migration inhibition when an active component according to the present invention is administered.

DETAILED DESCRIPTION OF THE INVENTION

According to the present invention, hydroxamic acid derivatives represented by formula (I):

(I)

(wherein $R^1$ represents a hydrogen atom, C1-8 alkyl, or C1-8 alkyl substituted with $-OR^2$; and $R^2$ represents a hydrogen atom or C1-8 alkyl, benzyl, or C1-8 alkyl substituted with C1-8 alkoxy) or nontoxic salts thereof are used.

The compound represented by formula (I) can be produced by the method described in WO 99/19296.

In the present invention, examples of the C1-8 alkyl include methyl, ethyl, propyl, butyl, pentyl, hexyl, heptyl and octyl groups and isomers thereof.

In the present invention, examples of the C1-8 alkyl substituted with $-OR^2$ include methyl, ethyl, propyl, butyl, pentyl, hexyl, heptyl and octyl groups substituted with one $-OR^2$ and isomers thereof.

In the present invention, examples of the C1-8 alkyl substituted with C1-8 alkoxy include methyl, ethyl, propyl, butyl, pentyl, hexyl, heptyl and octyl groups and isomers thereof substituted with one group selected from methoxy, ethoxy, propoxy, butoxy, pentyloxy, hexyloxy, heptyloxy and octyloxy groups and isomers thereof.

In the present invention,

is a bond showing an isomer due to the presence of an asymmetric carbon or a mixture thereof, specifically showing:

which indicates that it is bound to the front side of the sheet;

which indicates that it is bound to the opposite side of the sheet; or a mixture thereof.

In the present invention, all of these isomers are included, unless otherwise indicated. For example, straight chain and branched chain groups are included in alkyl groups and alkoxy groups. In addition, all of isomers due to the presence of asymmetric carbon and the like (R and S configrations, $\alpha$ and $\beta$ configrations, enantiomers and diastereomers), optically active substances having optical rotatory power (D, L, d and I isomers), polar compounds by chromatography separation (more polar compounds and less polar compounds), equilibrium compounds, mixtures thereof

having optional ratio and racemic mixtures are included in the present invention.

In the present invention, all of the groups represented by $R^1$ are preferred, but more preferred is a hydrogen, C1-4 alkyl, or C1-4 alkyl substituted with one $-OR^2$.

In the present invention, all of the groups represented by $R^2$ are preferred, but more preferred is a hydrogen atom, C1-4 alkyl, benzyl, or C1-4 alkyl substituted with one C1-4 alkoxy.

Specific examples of the compound used in the present invention include:

N-hydroxy-5-hydroxy-2(S)-methyl-4(S)-(4-phenoxybenzoyl)aminopentaneamide,
N-hydroxy-5-methoxymethyloxy-2(S)-methyl-4(S)-(4-phenoxybenzoyl)aminopentaneamide,
N-hydroxy-5-ethoxymethyloxy-2(S)-methyl-4(S)-(4-phenoxybenzoyl)aminopentaneamide,
N-hydroxy-5-ethoxymethyloxy-2(R)-methyl-4(R)-[N-(4-phenoxyphenylcarbonyl)amino]pentaneamide,
N-hydroxy-5-ethoxymethyloxy-2(R)-methyl-4(S)-[N-(4-phenoxyphenylcarbonyl)amino]pentaneamide,
N-hydroxy-5-benzyloxymethyloxy-2(S)-methyl-4(S)-(4-phenoxybenzoyl)aminopentaneamide, and
N-hydroxy-5-(2-methoxyethoxy)methyloxy-2(S)-methyl-4(S)-(4-phenoxybenzoyl)aminopentaneamide; and
nontoxic salts thereof.

The compounds used in the present invention may be converted into the corresponding salts. Non-toxic salts and water-soluble salts are preferred.

Examples of suitable salts include salts of alkali metals (e.g., potassium, sodium, *etc.*), salts of alkaline earth metals (e.g., calcium, magnesium, *etc.*), and ammonium salts, salts of pharmaceutically acceptable organic amines (e.g., tetramethylammonium, triethylamine, methylamine, dimethylamine, cyclopentylamine, benzylamine, phenethylamine, piperidine, monoethanolamine, diethanolamine, tris(hydroxymethyl)amine, lysine, arginine, N-methyl-D-glucamine, *etc.*).

The compounds used in the present invention and salts thereof may be converted into the corresponding hydrates by conventional manner.

BEST MODE FOR CARRYING OUT THE INVENTION

Efficacy of the compound of the present invention on diseases due to a constrictive vascular lesion was proved, e.g., by the following tests.

Test Methods:

Preparation of hamster blood vessel stricture model

Male hamsters (Gold, SLC, Japan; 40 to 50 g) were allowed to feed on 0.5% cholesterol feed for 4 weeks to increase the LDL value to 4 to 6 times higher than that of normal hamsters. Each hamster was anesthetized with pentobarbital (50 mg/kg), and its carotid inner membrane was peeled using 2FG catheter (Portex) whose tip had been roughly shaved.

(1) Effect to inhibit thickening of blood vessel

After the carotid inner membrane injury, the animals were observed for 2 weeks. As a drug to be tested, N-hydroxy-5-ethoxymethyloxy-2(S)-methyl-4(S)-(4-phenoxybenzoyl)aminopentaneamide represented by a formula (II):

(II)

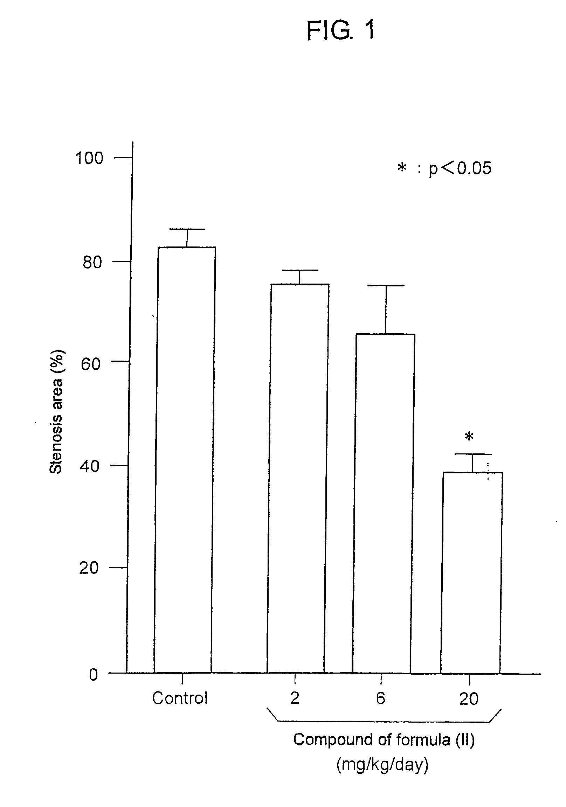

was orally administered once before the carotid inner membrane injury, and then its oral administration was continued

twice a day for 2 weeks (2, 6 or 20 mg/kg/day). On the 15th day after the blood vessel injury, the damaged blood vessel was took out under pentobarbital anesthesia (50 mg/kg) to prepare a frozen sample of about 2 mm. This section was divided into 3 pieces, and each of them was cut in round slices at 100 μm intervals and subjected to hematoxylin eosin (H-E) staining. Using a computer image analyzer (NIH Image), inhibition of thickening area was calculated as a ratio of the thickened area against the intra-vascular area (steneosisi area %). The measurement was carried out three times for each section, and their average value was obtained. The results are shown in Fig. 1.

(2) Effect to inhibit growth of blood vessel smooth muscle cells

The drug to be tested, N-hydroxy-5-ethoxymethyloxy-2(S)-methyl-4(S)-(4-phenoxybenzoyl)aminopentaneamide represented by the above formula (II), was orally administered once before the blood vessel inner membrane injury, and then it was orally administered twice a day (20 mg/kg/day). 5-Bromo-2-deoxy-uridine (BrdU: 50 mg/kg) was injected by subcutaneous injection 1, 8, 16 or 24 hours before taking out the inner membrane-injured carotid from each hamster.

After the carotid injury, the injured blood vessels were taken out on the 1st and 7th days to prepare frozen samples. BrdU positive cells were stained with a mouse monoclonal antibody (SIGMA) and then with a peroxidase-conjugated goat anti-mouse IgG antibody and detected with diaminobenzidine (DAB). In order to confirm the total number of cells, each section was stained with hematoxylin. The BrdU labeling index of intermediate membrane and newly formed inner membrane was calculated by the following formula:

BrdU labeling index

= [the number of positive nuclei stained with DAB

/ the number of total nuclei stained with hematoxyline] x 100

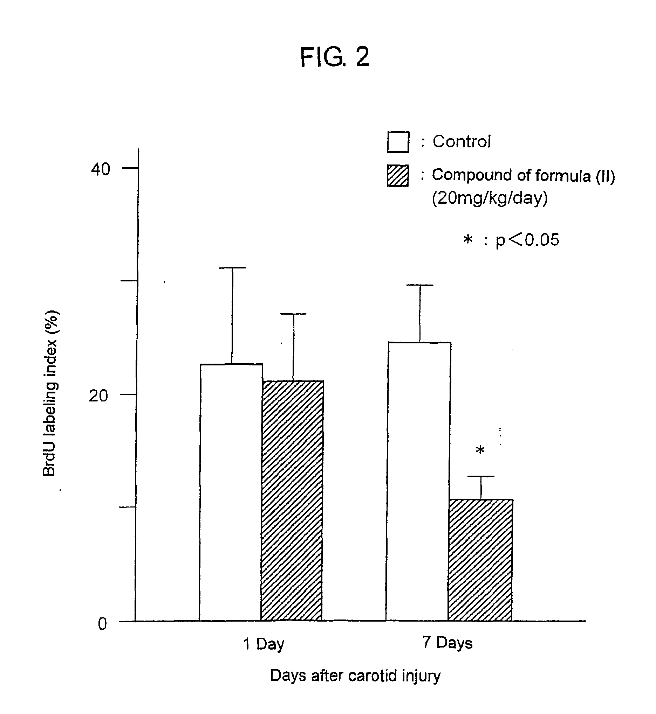

From the results shown in Fig. 2, it can be understood that the growth was significantly inhibited on the 7th day after the injury.

(3) Effect to inhibit migration of blood vessel smooth muscle cells

Administration of the drug to be tested and BrdU was carried out in the same manner in the above (2).

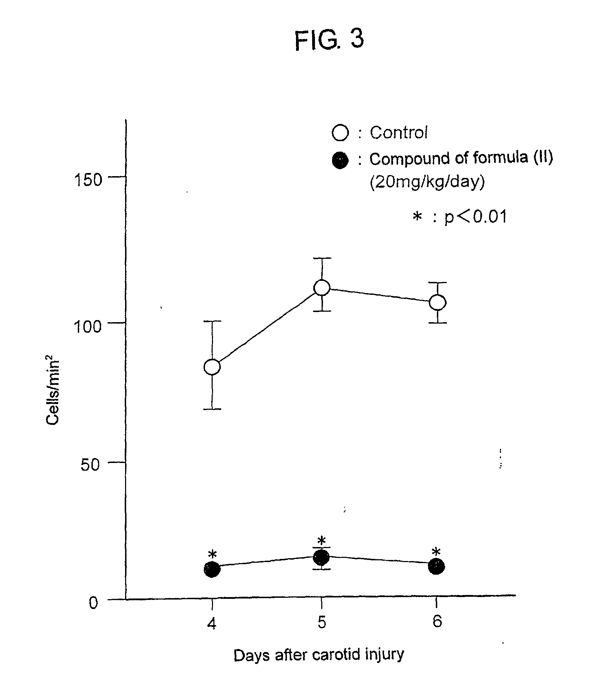

The injured blood vessels were taken out from the hamsters on 3, 5 and 7th days after the carotid injury. Each blood vessel was washed with PBS and put into 0.3% hydrogen peroxide/cold methanol solution. The sample was incubated for 45 minutes in 5% goat serum diluted with 1% BSA-containing PBS to prevent nonselective linkage of protein. This was incubated for 60 minutes in 1% BSA-containing PBS using a histone H1 monoclonal antibody. Thereafter, each section was incubated for 45 minutes using 1% goat anti-mouse IgG antibody diluted with 1% BSA-containing PBS. By measuring the number of histone H1 (+) cells (cells/$mm^2$), migration of smooth muscle cells from intermediate membrane to inner membrane was evaluated. The results are shown in Fig. 3. It can be understood from Fig. 3 that the number of smooth muscle cells on the surface of inner membrane is significantly reduced in comparison with the control group.

Industrial Applicability

Based on the above results, it was found that the compound represented by formula (I) inhibits migration and growth of blood vessel smooth muscle cells in a blood vessel stricture model at the time of high cholesterol feed loading and it also inhibits actual blood vessel thickening formation very potently. Thus, since the compound represented by formula (I) has an activity to inhibit stricture of blood vessels, it can be judged that the compound is effective for treating and/or preventing diseases due to a constrictive vascular lesion.

Toxicity:

The toxicity of the compounds of the present invention is very low and therefore, the compounds may be considered safe for pharmaceutical use. For example, the minimal lethal dose by single oral administration of N-hydroxy-5-ethoxymethyloxy-2(S)-methyl-4(S)-(4-phenoxybenzoyl)aminopentanamide using rats was 2000 mg/kg.

Application for Pharmaceuticals:

The hydroxamic acid derivatives represented by formula (I) used in the present invention having a potent inhibitory activity against matrix metalloproteinase, for example, gelatinase, stromelysin or the like, and non-toxic salts thereof are useful for treatment and/or prevention of diseases due to a constrictive vascular lesion in animals including human beings, especially human beings.
When the compounds of present invention, non-toxic salts thereof, acid addition salts and hydrates thereof are used for the above object, they are normally administered systemically or locally and orally or parenterally.
The doses to be administered are determined depending on, for example, age, body weight, symptom, therapeutic effect, administration route, duration of the treatment and the like. Generally, 1 mg to 1000 mg per adult is orally administered once to several times per day, or 0.1 mg to 100 mg per adult is parenterally administered (preferably by intravenous administration) once to several times per day, or continuously administered from vein for 1 to 24 hours per day.
Since the dose changes depending on various conditions as described above, there are cases in which doses lower than or greater than the above ranges may be used.
The compound of the present invention may be administered as solid forms for oral administration or liquid forms for oral administration, injections, external preparations or suppositories for parenteral administration, or the like.
Solid forms for oral administration include compressed tablets, pills, capsules, dispersible powders, granules and the like. Also, they include collutories, sublingual tablets and the like which are inserted into and adhered to the oral cavity. Capsules include hard capsules and soft capsules.
In such solid forms for oral administration, one or more of the active compound(s) may used as they are or mixed with vehicles (lactose, mannitol, glucose, microcrystalline cellulose, starch, *etc.*), binders (hydroxypropyl cellulose, polyvinylpyrrolidone, magnesium metasilicate aluminate, *etc.*), disintegrants (cellulose calcium glycolate, *etc.*), lubricants (magnesium stearate, *etc.*), stabilizing agents, solution adjuvants (glutamic acid, aspartic acid, *etc.*) and the like, and formulated in the usual manner. Also, if necessary, the solid forms may be coated with coating agents (sugar, gelatin, hydroxypropyl cellulose or hydroxypropylmethyl cellulose phthalate, *etc.*) or be coated with two or more films. Furthermore, capsules of absorbable materials such as gelatin are also included.
Liquid forms for oral administration include pharmaceutically acceptable solutions, suspensions, emulsions, syrups, elixirs and the like. In such liquid forms, one or more of the active compound(s) may be dissolved, suspended or emulsified into generally used diluent(s) (purified water, ethanol, a mixture thereof, *etc.*). Furthermore, liquid forms may include wetting agents, suspending agents, emulsifying agents, sweetening agents, flavoring agents, aroma, preservative, buffering agent and the like.
Injections for parenteral administration include solutions, suspensions, emulsions and solid forms which are dissolved or suspended into solvent(s) for injection immediately before use. In injections, one or more of the active compound(s) may be dissolved, suspended or emulsified into solvent(s). The solvents include distilled water for injection, physiological saline, vegetable oil, propylene glycol, polyethylene glycol, alcohol such as ethanol, and a mixture thereof. Furthermore, injections may contain stabilizing agents, solution adjuvants (glutamic acid, aspartic acid, POLYSORBATE 80 (registered trade mark), *etc.*), suspending agents, emulsifying agents, soothing agent, buffering agents, preservatives and the like. They may be sterilized at a final step, or may be prepared and compensated according to sterile methods. They may also be manufactured in the form of sterile solid forms such as freeze-dried products, which may be dissolved in sterile water or some other sterile diluent(s) for injection immediately before use.
Other forms for parenteral administration include liquids for external use, ointments and endermic liniments, inhalations, sprays, suppositories, pessaries for vaginal administration and the like which contain one or more of the active compound(s) and which are prepared by the usual manner.
Sprays may contain stabilizing agents such as sodium sulfate, isotonic buffers such as sodium chloride, sodium citrate and citric acid, and the like, in addition to the generally used diluents. The method for preparing the sprays are described in detail in the U.S. Patents 2,868,691 and 3,095,355.

Formulation Examples:

Formulation Example 1

The following components were mixed in the usual manner and punched out to obtain 100 tablets each containing 50 mg of the active ingredient.

- N-hydroxy-5-ethoxymethyloxy-2(S)-methyl-4(S)- 5.0 g (4-phenoxybenzoyl)aminopentanamide
- Carboxymethylcellulose calcium (disintegrating agent) 0.2 g
- Magnesium stearate (lubricating agent) 0.1 g

- Microcrystaline cellulose 4.7 g

Formulation Example 2

The following components were mixed in the usual manner. Then, the resulting solution was sterilized in the usual manner, packed into ampoules at 5 ml and freeze-dried in the usual manner to obtain 100 ampoules each containing 20 mg of the active ingredient.

- N-hydroxy-5-ethoxymethyloxy-2(S)-methyl-4(S)- 2.0 g (4-phenoxybenzoyl)aminopentanamide
- Mannitol 20 g
- Distilled water 500 ml

## Claims

**1.** An agent for treating and/or preventing diseases due to a constrictive vascular lesion, which comprises, as an active ingredient, a hydroxamic acid derivative represented by formula (I):

(I)

wherein $R^1$ represents a hydrogen atom, C1-8 alkyl, or C1-8 alkyl substituted with $-OR^2$; and $R^2$ represents a hydrogen atom, C1-8 alkyl, benzyl, or C1-8 alkyl substituted with C1-8 alkoxy, or a nontoxic salt thereof.

**2.** The agent for treating and/or preventing diseases due to a constrictive vascular lesion according to claim 1, wherein the compound is

N-hydroxy-5-hydroxy-2(S)-methyl-4(S)-(4-phenoxybenzoyl)aminopentaneamide,
N-hydroxy-5-methoxymethyloxy-2(S)-methyl-4(S)-(4-phenoxybenzoyl)aminopentaneamide,
N-hydroxy-5-ethoxymethyloxy-2(S)-methyl-4(S)-(4-phenoxybenzoyl)aminopentaneamide,
N-hydroxy-5-ethoxymethyloxy-2(R)-methyl-4(R)-[N-(4-phenoxyphenylcarbonyl)amino]pentaneamide,
N-hydroxy-5-ethoxymethyloxy-2(R)-methyl-4(S)-[N-(4-phenoxyphenylcarbonyl)amino]pentaneamide,
N-hydroxy-5-benzyloxymethyloxy-2(S)-methyl-4(S)-(4-phenoxybenzoyl)aminopentaneamide, or
N-hydroxy-5-(2-methoxyethoxy)methyloxy-2(S)-methyl-4(S)-(4-phenoxybenzoyl)aminopentaneamide; or
a nontoxic salt thereof.

**3.** An agent for treating and/or preventing restenosis after percutaneous transluminal coronary angioplasty, which comprises, as an active ingredient, a compound represented by formula (I) of claim 1 or a nontoxic salt thereof.

FIG. 1

100
80
60
40
20
0
Stenosis area (%)
* : p<0.05
*
Control
2
6
20
Compound of formula (II)
(mg/kg/day)

FIG. 2

: Control
: Compound of formula (II)
(20mg/kg/day)
* : p<0.05
40
20
0
BrdU labeling index (%)
*
1 Day
7 Days
Days after carotid injury

FIG. 3

○ : Control
● : Compound of formula (II)
(20mg/kg/day)
* : p<0.01
150
100
50
0
Cells/min²
4
5
6
Days after carotid injury

## INTERNATIONAL SEARCH REPORT

International application No.
PCT/JP01/02946

A. CLASSIFICATION OF SUBJECT MATTER
Int.Cl$^7$ A61K31/166, A61P9/10, 9/08

According to International Patent Classification (IPC) or to both national classification and IPC

B. FIELDS SEARCHED

Minimum documentation searched (classification system followed by classification symbols)
Int.Cl$^7$ A61K31/166, A61P9/10, 9/08

Documentation searched other than minimum documentation to the extent that such documents are included in the fields searched

Electronic data base consulted during the international search (name of data base and, where practicable, search terms used)
CA(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | WO, 99/19296, A1 (ONO PHARMACEUTICAL CO., LTD.), 22 April, 1999 (22.04.99), (especially, examples 49(39), 49(116), 49(255), 58(2), 71) & AU, 9894580, A & EP, 1024134, A1 & ZA, 9809113, A & BR, 9812807, A & NO, 2000001813, A | 1-3 |
| Y | Nobuya Zempo, et al., "Regulation of Vascular Smooth Muscle Cell Migration and Proliferation In Vitro and in Injured RatArteries by a Synthetic Matrix Metalloproteinase Inhibitor, "Arterioscler., Thromb., Vasc. Biol., Vol.16, No.1 (1996), pp.28-33 | 1-3 |
| Y | Michelle P. Bendeck, et al., "Inhibition of Matrix Metalloproteinase Activity Inhibits Smooth Muscle Cell Migration but Not Neointimal Thickening After Arterial Injury," Circ Res. Vol.78, No.1 (1996), pp.38-43 | 1-3 |
| Y | Kyle Northcote Cowan, et al., "Elastase and matrix metalloproteinase inhibitors induce regression, and tenascin-C antisense prevents progression, of | 1-3 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 June, 2001 (08.06.01) | 19 June, 2001 (19.06.01) |

| Name and mailing address of the ISA/ | Authorized officer |
|---|---|
| Japanese Patent Office | |
| Facsimile No. | Telephone No. |

Form PCT/ISA/210 (second sheet) (July 1992)

INTERNATIONAL SEARCH REPORT

International application No. PCT/JP01/02946

C (Continuation). DOCUMENTS CONSIDERED TO BE RELEVANT

| Category* | Citation of document, with indication, where appropriate, of the relevant passages | Relevant to claim No. |
|---|---|---|
| | vascular disease," J. Clin. Invest., Vol.105, No.1 (January 2000) pp.21-34 | |

Form PCT/ISA/210 (continuation of second sheet) (July 1992)